# EUROPÄISCHE PATENTSCHRIFT

(11) **EP 1 412 564 B1**
(45) Veröffentlichungstag und Bekanntmachung des Hinweises auf die Patenterteilung: **22.07.2009**
(21) Anmeldenummer: 02747228.1
(22) Anmeldetag: 20.06.2002
(51) Int. Cl.: D01D 5/24, D01F 8/08, B01D 69/08, B01D 71/42

(54) **ZWEISCHICHT-HOHLMEMBRAN FÜR BIOREAKTORANWENDUNGEN**
DOUBLE-LAYERED-HOLLOW MEMBRANE FOR BIOREACTOR APPLICATIONS
MEMBRANE BICOUCHE A FIBRES CREUSES CONCUE POUR DES APPLICATIONS FAISANT APPEL A UN BIOREACTEUR

(30) Priorität: 16.07.2001 DE 10134447
(43) Veröffentlichungstag der Anmeldung: 28.04.2004
(73) Patentinhaber: GKSS-Forschungszentrum Geesthacht GmbH, 21502 Geesthacht (DE)
(72) Erfinder: ALBRECHT, Wolfgang, 14513 Teltow (DE); WEIGEL, Thomas, 14557 Wilhelmshorst (DE); GROTH, Thomas, 10439 Berlin (DE); SEIFERT, Barbara, 10249 Berlin (DE); HILKE, Roland, 14513 Teltow (DE); PAUL, Dieter, 14532 Kleinmachnow (DE)
(74) Vertreter: Seemann, Ralph
(86) Internationale Anmeldenummer: PCT/DE2002/002249
(87) Internationale Veröffentlichungsnummer: WO 2003/008677

(56) Entgegenhaltungen:
- EP-A- 1 177 829
- WO-A-02/36327
- DE-A- 2 321 460
- DE-A- 2 705 735
- DE-A- 4 021 052
- F. FEY-LAMPRECHT ET AL: "Development of membranes for the cultivation of kidney epithelial cells" BIOMATERIALS, Bd. 21, 2000, Seiten 183-192, XP002219866
- VON SENGBUSCH G.: 'Blood - Material Interaction', 1998, INTERNATIONAL FACULTY FOR ARTIFICIAL ORGANS (INFA) * Seite 79 - Seite 89 *
- GURLAND H.J. ET AL NEPHROLOGY DIALYSIS TRANSPLANTATION Bd. 9, Nr. 2, Seiten 4 - 10
- ZELTNER: "Technische Normen für Medizinprodukte, Medizinprodukteverordnung (MepV)" 19. Oktober 1999 (1999-10-19), BEKANNTMACHUNGEN DER DEPARTEMENTE UND DER ÄMTER/CH * Seite 8579 *
- "EN ISO 10993-4:2002"
- RAHMAN K.H. ET AL ELECTROCHIMICA ACTA Bd. 50, 2004, Seiten 633 - 638
- HEILMANN K. ET AL JOURNAL OF BIOTECHNOLOGY Bd. 115, 2005, Seiten 291 - 301
- "IUPAC Recommendations on Macromolecular (Polymer) Nomenclature, www.iupac.org" 18. Mai 2001 (2001-05-18), IUPAC Seiten 1-17, 130-145

## Beschreibung

Die Erfindung betrifft eine Trägermembran für Biohybridorgane und Biohybridreaktoren mit einer Zweischicht-Membranwand, deren Schichten aus unterschiedlichen Polymeren bzw. Polymergemischen auf der Basis von Polyacrylnitril bestehen und deren eine Oberfläche blutkompatibel ist, während deren andere Oberfläche gewebekompatibel ist, ein Verfahren zur Herstellung dieser Membran und die Verwendung dieser Trägermembran als Trägermembran für Biohybridorgane.

Es ist bereits bekannt, Membrane zur extrakorporalen Detoxifikation von Blut bei Organversagen, beispielsweise der Niereninsuffizienz, einzusetzen. Bei dieser heute weltweit in den Klinken angewandten Behandlung permeieren Toxine entsprechend der Porengröße der zur Anwendung gebrachten Dialysemembran und der Molekülgröße der zu entfernenden Stoffe aus dem Blut des Patienten in eine Spüllösung, das Dialysat; dieser Vorgang wird hier als "passiver" Transport bezeichnet, der auf den Konzentrationsunterschieden der Inhaltsstoffe in beiden Flüssigkeiten entsprechend den Gesetzen der Diffusion basiert.

In der natürlichen Niere erfolgt die Entfernung der Uremietoxine in erster Stufe ebenfalls auf Basis eines passiven Prozesses, bei dem der Primärharn entsteht. Im Gegensatz zur künstlichen Niere ist jedoch diesem passiven Ultrafiltrationsprozess ein aktiver Prozess nachgeschaltet, in dem Inhaltsstoffe des Primärharnes und vor allem Wasser entgegen dem Konzentrationsgradienten vom Primärharn zurück ins Blut transportiert werden. Ein derartiger aktiver Prozess ist bei der künstlichen Niere beim gegenwärtigen Entwicklungsstand der Membrantrenntechnik nur durch den Einsatz vitaler und gewebeartig organisierter Zellschichten realisierbar. Gleiches gilt für andere Organe, beispielsweise das Pankreas und die Leber. Mittels Porenmembranen können somit lediglich die passiven Filtrationsfunktion und evtl. die sorptive Funktion der für die Entgiftung zuständigen Organe unterstützt bzw. ersetzt und somit auch therapeutisch nutzbar gemacht werden.

Es hat nun nicht an Versuchen gefehlt, um den aktiven Transport vitaler Zellen gezielt zur Unterstützung von Dysfunktionen kranker Organe einzusetzen. Die bisher erzielten Wirkungen können therapeutisch jedoch kaum angemeldet werden. Die Ursachen dafür sind vielfältig.

Angesichts der klinischen Relevanz hat es jedoch nicht an Versuchen gefehlt, geeignete Membranträger mit einem für den speziellen Anwendungsfall erforderlichen Eigenschaftsprofil zu entwickeln. Im Prinzip können die bisher bekannten Membranträger in vier unterschiedliche Grundtypen klassifiziert werden, die sich insbesondere in den Membran-und/oder Queilungseigenschaften unterscheiden:
1. Hochquellende Gelmembrane mit Membranfunktion (z. B. ASAIO J. 39 (1993), M261 - M267; Biomaterials 16 (1995), 753 - 759) bzw. mit Gel gefüllte Porenmembrane (z. B. WO 97/17129).
2. Praktisch nicht oder moderat quellende Porenmembrane mit Membranfunktion in unfunktionalisierter (WO 95/21911, WO 96/40871, US 5837234) oder funktionalisierter Form (US 5720969), wobei das biochemische Verhalten hinsichtlich Blut- und Gewebeverträglichkeit in einer Polymerzusammensetzung des Membranbildners entsprechend den Erfordernissen optimiert sein kann, man vergleiche DE 100 30 307.2.
3. 3D-Matrixmaterialien ohne Trennfunktion (GB 2 187 447, WO 97/12960) mit extrem grobporiger Struktur (10-100µm).
4. Trägermaterialien, die mittels Techniken der Mikroelektronik, wie Dünnfilmablagerung, Photolithographie und/oder Ätzung hergestellt wurden (z. B. US 5 651 900, US 5 798 042, US 5 893 974, US 5 938 923, US 5 948 255, US 5 985 328).

Allen diesen Entwicklungen ist gemein, dass das Anforderungsprofil einer Trägermembran für deren Einsatz in Biohybridorganen nur partiell erfüllt wird: Gelmembranen oder gelgefüllte Membranen bewirken eine ungenügende Adhärenz für adhäsionsabhängige Zellen und bis heute einen ungenügenden Stoffaustausch. Nicht oder nur moderat quellende Porenmembranen weisen keine optimalen Eigenschaften hinsichtlich Blut- oder Gewebeverträglichkeit auf. Auch eine optimierte Polymerzusammensetzung realisiert nur einen Kompromiss zwischen beiden Kenngrößen; die Membranbildung nach einem Phaseninversionsprozess ist oft ungenügend. 3D-Matrixmaterialien besitzen grundsätzlich keine immunisolierende Wirkung, was den Einsatz nicht verfügbarer Patientenzellen erfordert. Die Permeationseigenschaften der mittels Techniken der Mikroelektronik gefertigten Materialien sind ungenügend und die Herstellung größerer Trägermembranflächen verbietet sich aus Kostengründen.

Aufgabe der vorliegenden Erfindung ist es daher, eine Trägermembran bereitzustellen, die in Biohybridreaktoren eingesetzt werden kann und dabei kostengünstig herzustellen ist.

Gelöst wird diese Aufgabe durch eine Membran gemäß der Lehre der Ansprüche.

Zur Herstellung der Zweischicht-Membranwand der erfindungsgemäßen Membran werden zwei chemisch unterschiedliche Polymerlösungen eingesetzt. Die erste Polymerlösung enthält dabei ein blutkompatibles Polymer, das in einem Lösemittel gelöst wird. Die zweite Polymerlösung wird getrennt von der ersten Polymerlösung hergestellt und enthält ein gewebekompatibles Polymer. Auch dieses gewebekompatible Polymer wird in einem Lösemittel gelöst. Die Herstellung der beiden Polymerlösungen erfolgt dabei derart, dass beide Polymerlösungen in Form von Polymermischlösungen im homogenen Zustand verbleiben.

Das blutkompatible Polymer ist das Polyacrylnitril-Copolymer ANNVP 20 oder heparinisiertes ANAPMA oder eine Mischung von ANNVP 20 mit Polyacrylnitril. Das gewebekompatible Polymer ist das Polyacrylnitril-Copolymer ANNVP 5 oder eine Mischung von ANNVP 5 und Polyacrylnitril. Die Abkürzungen ANNVP und ANAPMA bezeichnen in diesem Zusammenhang die Mischpolymere P(AN-co-NVP) und P(AN-co-APMA) nach der IUPAC-Konvention. Dabei bezeichnet ANNVP 5 ein P(AN-co-NVP)-Mischpolymer mit 6,1 Mol-% NVP-Anteil, ANNVP 20 ein P(AN-*co*-NVP)-Mischpolymer mit 18,0 Mol-% NVP-Anteil und ANAPMA ein heparinisiertes P(AN-co-APMA)-Mischpolymer mit 3,8 Mol-% APMA-Anteil.

Als Lösemittel für die Herstellung der beiden Polymerlösungen wird vorzugsweise das gleiche Lösemittel eingesetzt. Es ist auch möglich, Lösemittelgemische zur Anwendung zu bringen, wobei in diesem Falle vorzugsweise mindestens ein Bestandteil der zur Anwendung gebrachten Lösemittelgemische für beide Polymerlösungen der gleiche ist.

Die beiden oben beschriebenen, zur Herstellung der Membranwand dienenden Polymermischlösungen werden in einer Düse eines Extruders miteinander unter Bildung eines schichtartigen Polymerlösungsverbundes in Kontakt gebracht und gleichzeitig in dieser Form als Polymerlösungsverbund aus der Düse des Extruders extrudiert. Der Polymerlösungsverbund wird dabei in ein Koagulationsbad extrudiert und einer Phaseninversion unterzogen. Die gebildete Zweischicht-Membran wird dann abschließend auf per se bekannte Weise von allen nichtmembran-bildenden Bestandteilen zumindest teilweise befreit und erforderlichenfalls nachbehandelt, beispielsweise thermisch stabilisiert und präpariert sowie getrocknet und konfektioniert.

Vorzugsweise wird zwischen die Extrusion und das Koagulationsbad eine Luftstrecke dazwischen geschaltet.

Nach einer insbesondere bevorzugten Ausführungsform stellt die erfindungsgemäße Membran eine Zweischicht-Hohlmembran dar. Diese ist dadurch erhältlich, dass die beiden oben beschriebenen Polymerlösungen in Form von Polymermischlösungen unter Verwendung von Mehrkanalhohlkerndüsen entsprechend der DE 100 54 591.2, bei denen die die Zweischicht-Membranwand bildenden Polymerlösungen bereits in der Spinndüse unter Verlassen von derselben kontaktiert und in dieser Form als Polymerlösungsverbund gemeinsam zum Ausgang der Spinndüse transportiert werden.

Vorzugsweise wird dieser Polymerlösungsverbund am Ausgang der Spinndüse durch die gleichzeitige Extrusion eines den Hohlkern bildenden Lumenfüllers in seiner Form stabilisiert. Die weitere Bearbeitung erfolgt dann wie oben beschrieben.

Aus der Literatur ist bekannt, welche Polymere blutkompatibel bzw. gewebekompatibel sind. Auf Basis dieser Literaturdaten kann daher eine ausreichend gesicherte Auswahl der Polymere erfolgen. Anhand von einfachen Untersuchungen kann dann festgestellt werden, ob das blutkompatible bzw. gewebekompatible Polymer in dem zur Anwendung gebrachten Lösemittel oder Lösemittelgemisch derart gelöst werden kann, dass die resultierende Polymermischlösung ein homogenes Erscheinungsbild besitzt. Es hat sich jedoch gezeigt, dass Copolymere mit einem gemeinsamen Comonomer oft homogene Polymermischlösungen ergeben. Vorzugsweise ist die erfindungsgemäße Membran daher dadurch erhältlich, dass als Polymer für die Herstellung der Membranwand ein Polymergemisch aus einem blutkompatiblen bzw. gewebekompatiblen Polymer und einem Copolymer bzw. zusätzlichen Polymer eingesetzt wird. Insbesonders bevorzugt weisen dabei beide Polymerlösungen, die zur Herstellung der Zweischicht-Membranwand dienen, neben dem jeweiligen blutkompatiblen bzw. gewebekompatiblen Polymer gleichzeitig ein weiteres Polymer auf, das in beiden Polymerlösungen das gleiche ist bzw. das chemisch gleicht ist. Dies erleichtert die Herstellung von homogenen Polymermischlösungen. Zudem kann dieses zusätzliche, gemeinsame Polymer auch die Verträglichkeit verbessern, sowie gleichzeitig das Membranbildungsverhalten und das Verhalten bei thermischer Belastung vorteilhaft beeinflussen. So gelingt es, bei Verwendung derartiger Polymermischlösungen als die die Membranwand bildenden Polymerlösungen Zweischicht-Membranwände hoher Strukturintegrität zu formen. Der Massenteil an blut- bzw. gewebeverträglichen Polymeren und dem gemeinsamen Polymer im Gesamtpolymeranteil der jeweiligen Polymerlösungen beträgt dabei vorzugsweise 10 - 90 % und insbesondere bevorzugt 40 - 60 %.

Die Konzentration an Polymer in den einzelnen, die Membranwand bildenden Polymerlösungen kann in weiten Grenzen variiert werden und beträgt vorzugsweise 10 - 30 %. Durch die Variation dieser Konzentration und der Extrusionsbedingungen bzw. Spinnbedingungen kann das Trennverhalten der Zweischicht-Membranwand uns somit der erfindungsgemäßen Membran variiert und den jeweiligen Anforderungen nach Immunisolation der entsprechenden Zellart in entsprechenden Biohybridsystemen angepasst werden. Diese Polymere werden in den für die Polymere dem Fachmann bekannten Lösemittel bzw. Lösemittelgemischen gelöst.

Die Erfindung wird im folgenden anhand von Hohlmembranen näher erläutert, wobei dies jedoch nur aus Gründen der einfacheren Darstellbarkeit erfolgt und die beschriebenen Maßnahmen auch entsprechend oder analog bei Flachmembranen angewandt werden können.

Zur Herstellung der erfindungsgemäßen Membran in Form einer Hohlmembran werden vorzugsweise die beiden die Membranwand bildenden Polymerlösungen unter Verwendung der in der DE 100 54 591.2 beschriebenen Mehrkanalhohlkerndüse bereits in der Verformungsvorrichtung schichtartig zu einem Polymerverbund kontaktiert und als Polymerlösungsverbund gemeinsam aus der Spinndüse extrudiert. Am Ausgang der Düse wird der Polymerlösungsverbund durch die gleichzeitige Extrusion eines Lumenfüllers stabilisiert und ggf. über eine Luftstrecke in ein auf die verformten Polymerlösungen koagulierend wirkendes Medium geführt, in dem der geformte Polymerlösungsverbund als zweischichtige Hohlfadenmembran durch Phaseninversion verfestigt wird.

Der weitere Ablauf entspricht einem dem Fachmann bekannten Fertigungsprozess zur Herstellung einer Hohlmembran, bei dem die Hohlmembran ggf. verstreckt, gewaschen, ggf. thermisch stabilisiert und präpariert und abschließend getrocknet und konfektioniert wird.

Im Gegensatz zur "einschichtigen" Hohlmembran sind in der ggf. anzuwendenden thermischen Stabilisierung einer zweischichtigen Hohlmembran die Temperaturen dieser Stabilisierung derart auszuwählen, dass diese Temperatur kleiner als der Glasumwandlungspunkt derjenigen Polymerschicht ist, die den kleineren Glasumwandlungspunkt aufweist. Vorteilhaft ist auch in diesem Fall der Zusatz eines gemeinsamen, in beiden Polymerlösungen vorhandenen Polymers, dessen Glasumwandlungspunkt größer als die Glasumwandlungspunkte des blut-und des gewebeverträglichen Polymers ist.

Charakteristisch für die erfindungsgemäße zweischichtige Membran ist somit einerseits ein bifunktioneller chemischer Aufbau, dem ein strukturell asymmetrischer Aufbau überlagert sein kann, und andererseits eine strukturelle Integrität der die Zweischicht-Membranwand bildenden Polymerschichten (Adhärenz der beiden Polymerschichten).

Die erfindungsgemäße, poröse Membran, insbesondere in Form einer porösen Hohlmembran kann vorzugsweise als Trägermembran in Biohybridorganen (Hybriden aus "passiver" Membranfunktion und "aktiver" Organzellfunktion) zur Unterstützung bzw. zum temporären Ersatz erkrankter Organe eingesetzt werden.

Die erfindungsgemäße, poröse Membran ist einerseits entsprechend dem Einsatzziel permeabel genug, dabei andererseits jedoch immunisolierend und sowohl hämo- als auch gewebeverträglich. Sie verfügt zudem über ein gezielt einstellbares Proteinabsorptionsverhalten und ist kostengünstig herzustellen.

Nach einer bevorzugten Ausführungsform ist bei einer erfindungsgemäßen Membran in Form einer Hohlmembran die innere Oberfläche blutkompatibel, während die andere, äußere Oberfläche gewebekompatibel ist.

Beide Membranschichten des Hohlmembranquerschnittes besitzen eine strukturelle Integrität, was sich in einer Adhärenz beider Schichten zu einer verbundenen Membran äußert. Diese Voraussetzung ermöglichen einerseits eine Langzeitvitalität und -funktion der i.a. adhäsionsabhängigen Zellen und deren Anordnung in sich polar organisierenden Gewerbeverbänden sowie andererseits eine Langzeithämokompatibilität, die für den Einsatz von Biohybridsystemen zu fordern ist. Durch Variation der Herstellungsbedingungen können die Trenneigenschaften der Zweischichthohlmembranen so gestaltet werden, dass eine organspezifische Immunisolation realisiert werden kann, so dass verfügbare allogene bzw. xenogene Zellen in einem Biohybridorgan eingesetzt werden können, ohne dass das Immunsystem des Patienten aktiviert wird. Patientenzellen sind somit nicht mehr erforderlich, um beim Einsatz von Biohybridsystemen eine Immunabwehr des menschlichen Körpers gegenüber dem Biohybridorgan auszuschließen. Insgesamt entstehen Hohlmembranen, die das komplexe Anforderungsprofil von Trägermembranen für deren Einsatz in Biohybridsystemen erfüllen.

Die Erfindung wird im folgenden anhand von bevorzugte Ausführungsformen erläuternden Beispielen und unter Bezug auf die Figuren näher erläutert.

Von den Figuren zeigen:
- Figur 1:: Eine rasterelektronenmikroskopische Aufnahme des Querschnittes der Zweischicht-Hohlmembran gemäß dem Beispiel 1.
- Figur 2:: Eine rasterelektronenmikroskopische Aufnahme des Querschnittes der Zweischicht-Hohlmembran gemäß dem Beispiel 1 bei stärkerer Vergrößerung.
- Figur 3:: Eine rasterelektronenmikroskopische Aufnahme des Querschnittes der Zweischicht-Hohlmembran gemäß dem Beispiel 2.
- Figur 4:: Eine rasterelektronenmikroskopische Aufnahme der Grenzschicht zwischen beiden Polymerschichten der Zweischicht-Hohlmembran gemäß dem Beispiel 2.
- Figur 5:: Eine rasterelektronenmikroskopische Aufnahme des Querschnittes der Zweischicht-Hohlmembran gemäß dem Beispiel 3.
- Figur 6:: Eine rasterelektronenmikroskopische Aufnahme der Grenzschicht zwischen beiden Polymerschichten der Zweischicht-Hohlmembran gemäß dem Beispiel 3.
- Figur 7:: Eine rasterelektronenmikroskopische Aufnahme von MDCK-Zellen, die 8 Tage in einem Faser-in-Faser-Bioreaktor zwischen einer äußeren Polysulfonfaser und einer inneren ANNVP 5-Faser kultiviert wurden.
- Figur 8:: Eine transmissionselektronenmikroskopische Aufnahme von MDCK-Zellen, die 8 Tage in einem Faser-in-Faser-Bioreaktor zwischen einer äußeren Polysulfonfaser und einer inneren ANNVP 5-Faser kultiviert wurden; deutlich sichtbar ist der enge Zellkontakt zur ANNVP 5-Faser (Vergrößerung 21.000 fach).
- Figur 9:: Eine transmissionselektronenmikroskopische Aufnahme von MDCK-Zellen, die 7 Tage in einem Faser-in-Faser-Bioreaktor zwischen einer äußeren Polysulfonfaser und einer inneren ANNVP 5-Faser kultiviert wurden; die Zellen haben einen guten Kontakt zur ANNVP 5-Faser und zahlreiche Mikrovillositäten (MV) sind ausgeprägt (Vergrößerung 7.100 fach).
- Figur 10:: Eine transmissionselektronenmikroskopische Aufnahme von MDCK-Zellen, die 7 Tage in einem Faser-in-Faser-Bioreaktor zwischen einer äußeren Polysulfonfaser und
einer inneren ANNVP 5-Faser kultiviert wurden; deutlich sichtbar sind der gute Zell-Zellkontakt (tight junctions - TJ) und die Ausprägung der Mikrovillositäten (MV, Vergrößerung 31.000 fach).

Nachstehend wird die Herstellung von zweischichtigen, porösen Hohlmembranen aus unterschiedlichen Acrylnitrilpolymeren und deren Charakteristika beschrieben. Zudem wird die Anwendung dieser erfindungsgemäßen Hohlmembranen als Trägermembran in Biohybridsystemen unter Verwendung von Nierenepithelzellen (MDCH-Zellen) erläutert. Als Acryinitrilpolymere wurden die folgenden Polymere mit den folgenden Charakteristika eingesetzt.

| Produkt | Comonomer | Gehalt [Mol-%] | Eigenschaft | Lage der Schicht | Her-steller |
|---|---|---|---|---|---|
| PAN* | ohne | - | verstärkend, stabilisierend | Zusatz innen und /oder außen | Hoechst |
| ANNVP 5 | NVP** | 6,1 | gewebekompatibel | außen | Lab-Produkt |
| ANNVP20 | NVP** | 18,0 | blutkompatibel | innen | Lab-Produkt |
| ANAPMA | APMA*** | 3,8 | funktionatisierbar | innen | Lab-Produkt |

| | | | | | |
|---|---|---|---|---|---|
| **•** - Polyacrylnitril; ** - N-Vinylpyrrolidon; *** - Aminopropylmethacrylamid; | | | | | |

Die gute Blutverträglichkeit des hier eingesetzten ANNVP 20-Copolymers wurde in der Patentanmeldung DE 100 30 307.2 beschrieben und dokumentiert. Die vorzügliche Gewebeverträglichkeit von Polyacrylnitril und dessen Copolymeren ANNVP 5 für Nierenepithelzellen wird in der Publikation "Morphological studies on the culture of kidney epithelial cells in a fiber-in-fiber bioreactor design using hollow fiber membranes" (F. Fey-Lamprecht, W. Albrecht, Th. Groth, Th. Weigel, U. Gross, J. Biomed.Mat.Res.) (eingereicht 2001) dokumentiert.

### Beispiel 1

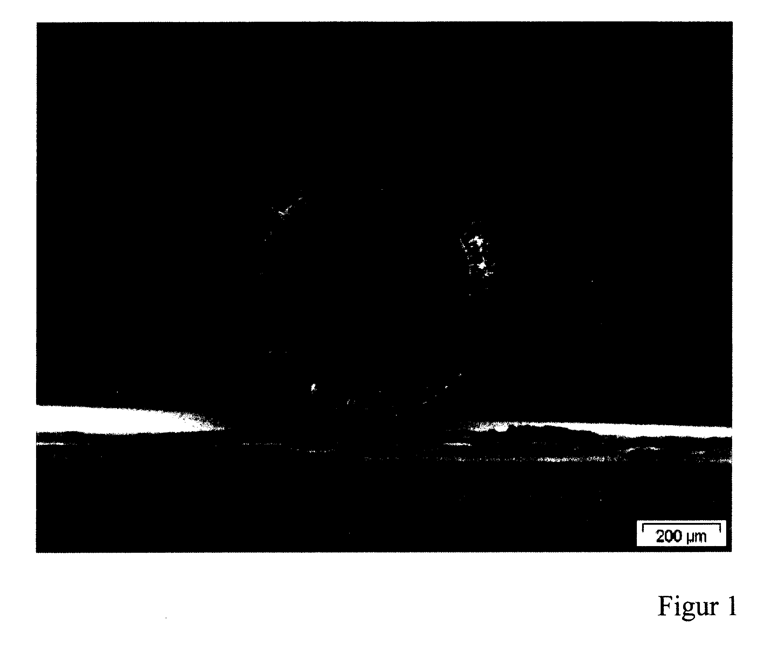

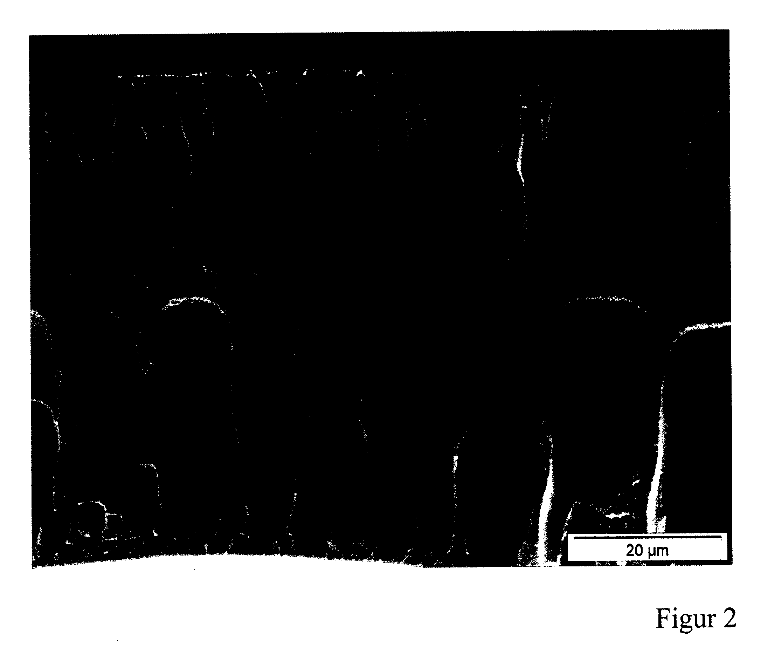

Es wurde eine Polymerlösung, bestehend aus 8 Teilen PAN, 8 Teilen ANNVP 5-Copolymer und 84 Teilen N,N-Dimethylformamid (DMF), durch Lösen der Polymere unter Rühren 2 h bei 80 °C hergestellt, wobei in einer ersten Stufe das PAN im Lösemittel gelöst und zu dieser Lösung die entsprechende Menge an AN-Copolymer zugegeben wurde. Nach Abkühlen wurde diese Polymerlösung unter Verwendung einer Filterkombination, bestehend aus Polyesterseparator (Sartorius)/Vliesstoff (Carl Freudenberg) filtriert, entgast und als Polymermischlösung der äußeren Membranschicht der Zweischichtmembran verwendet. In gleicher Weise wurde eine Lösung bestehend aus 8 Teilen PAN, 8 Teilen ANNVP 20-Copolymer und 84 Teilen DMF hergestellt, vorbereitet und als Polymermischlösung für die innere Polymerschicht verwendet. Als Lumenfüller und als Koagulationsbad wurde Wasser verwendet. Die Polymermischlösungen wurden unter Verwendung einer Mehrkanalhohlkerndüse der Dimensionen 190 µm/360 µm/560 µm/760 µm (Hohldorndurchmesser/innerer Schlitzdurchmesser/Durchmesse der Zusammenführungen beider Polymermischlösungen in der Düse/äußerer Schlitzdurchmesser) zum stabilisierten Polymerlösungsverbund verformt und direkt in ein Koagulationsbad extrudiert. Durch Einwirkung des Lumenfüllers und des Koagulationsbades wird der Polymerlösungsverbund fixiert und mit einer Geschwindigkeit von 8 m/min am Kopf des Fällbades abgezogen, mittels Waschwalze gewaschen und auf Spulen aufgenommen. In dieser Form erfolgt eine diskontinuierliche Waschung für mehr als 12 h. Anschließend wurde die Zweischicht-Hohlmembran kontinuierlich thermisch bei 98 °C für ca. 5 sec stabilisiert, bei 4 °C bei ca. 3 sec fixiert, mit 15 Gew.-%iger wässriger Glyzerinlösung präpariert, auf eine Haspel aufgenommen und bei Raumtemperatur getrocknet. Es entstand eine Zweischicht-Hohlmembran, deren Morphologie in den Figuren 1 und 2 wiedergegeben ist.

Es entstand eine erfindungsgemäße Zweischicht-Hohlmembran mit einer strukturellen Integrität über den Membranquerschnitt, aufgebaut aus einer blutverträglichen Innenschicht und einer gewebeverträglichen Außenschicht. Beide Polymerschichten sind vollständig miteinander verbunden. Trotz stärkerer Vergrößerung konnte keine Grenzfläche zwischen beiden Polymerschichten in den rasterelektronenmikroskopischen (REM)-Aufnahmen erkannt werden. Die unterschiedliche Form und Struktur der Fingerporen, die sich als Folge der unterschiedlichen Phaseninversion der beiden Polymerlösungen ergaben, belegt jedoch den Zweischichtaufbau des Membranquerschnittes. Die Verhältnisse beider Polymerschichten der Zweischichtmembran beiträgt hinsichtlich der Schichtdicke ca. 1.

Weitere charakteristische Daten dieser Hohlmembran sind im folgendem zusammengestellt:

| | |
|---|---|
| Innendurchmesser [µm] : | 508 |
| Gesamtwandstärke [µm] : | 78 |
| Wasserdurchlässigkeit [lm⁻²h⁻¹kPa⁻¹] : | 0.05 |

### Beispiel 2:

Es wurde entsprechend dem Beispiel 1 eine Zweischicht-Hohlmembran mit dem Unterschied ersponnen, dass die thermische Stabilisierung bei 30°C erfolgte.

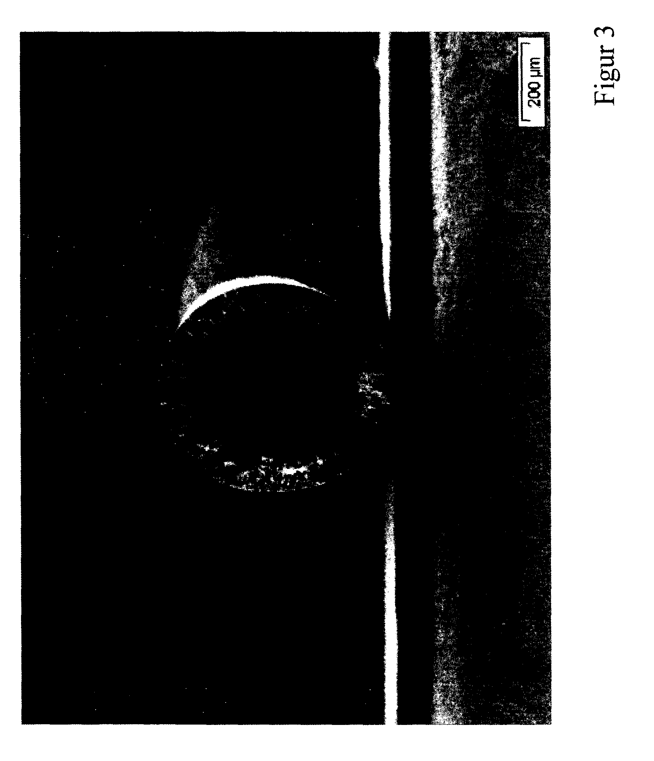

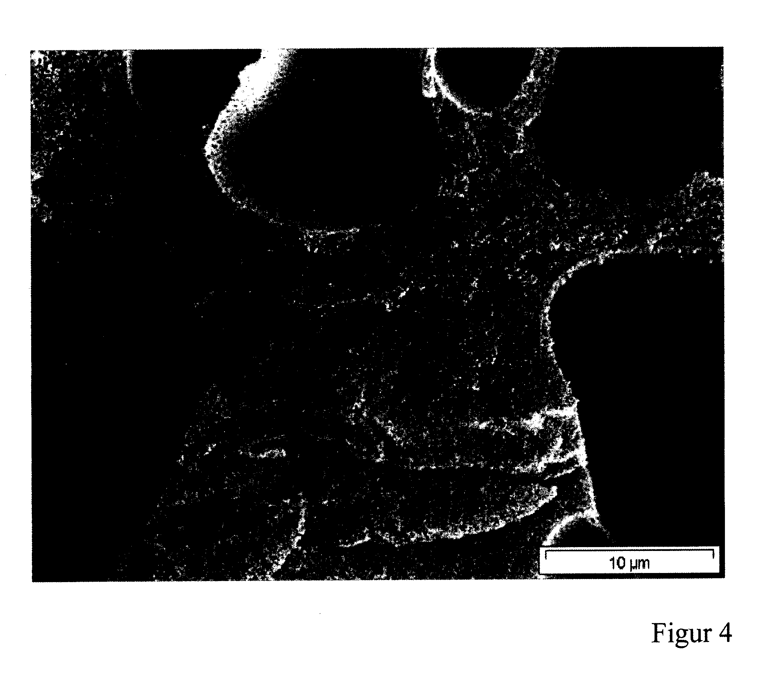

Die Morphologie dieser Zweischicht-Hohlmembran ist in Figur 3 und eine stark vergrößerte Abbildung der Grenzschicht zwischen beiden Polymerschichten dieser Membran ist in Figur 4 dargestellt.

Es entstand eine Zweischicht-Hohlmembran im Sinne dieser Erfindung mit einer strukturellen Integrität über den Membranquerschnitt, bestehend aus einer blutverträglichen Innenschicht und einer gewebeverträglichen Außenschicht. In Figur 4 ist die Grenzschicht zwischen beiden Polymerschichten angedeutet. Die strukturelle Integrität des Querschnittes ist so ideal, dass lediglich aus geringen Änderungen in der Morphologie die Grenzschicht im unteren Teil der Figur 4 vermutet werden kann. Die Gesamtstruktur belegt jedoch eindeutig den Aufbau der Zweischichthohlmembran aus zwei Polymerschichten. Das Verhältnis beider Schichtdicken beträgt ca. 1.

Weitere charakteristische Daten dieser Hohlmembran sind im folgendem zusammengestellt:

| | |
|---|---|
| Innendurchmesser [µm] : | 405 |
| Gesamtwandstärke [µm] : Gesamtwandstärke [µm] : | 87 |
| Wasserdurchlässigkeit [lm-²h-¹kPa-¹] : | 0,43 |
| Mittlerer Porendurchmesser [nm] : | 5,6 |
| Cut-off [kDa] : | 38,0 |

### Beispiel 3:

Entsprechend dem Beispiel 2 wurde eine Zweischicht-Hohlmembran mit dem Unterschied ersponnen, dass die Polymerlösung, die in der Zweischicht-Hohlmembran die innere Schicht bildet, aus 16 Teilen ANAPMA und 84 Teilen DMF bestand.

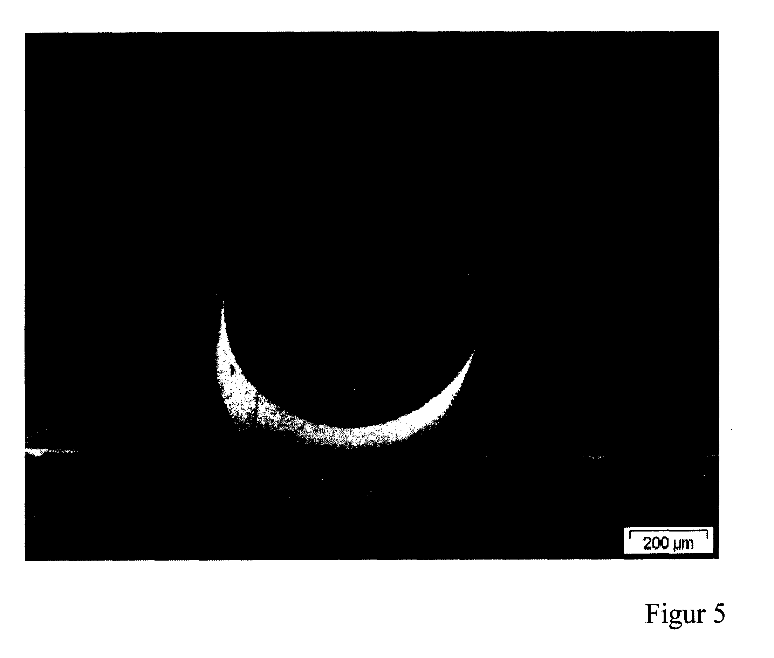

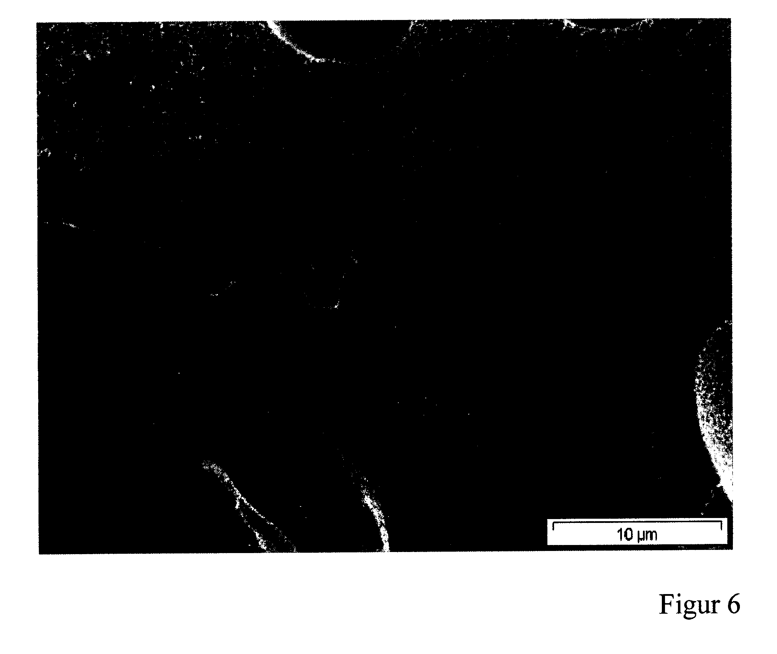

Die Morphologie dieser Zweischicht-Hohlmembran ist in Figur 5 und die der Grenzfläche zwischen beiden Polymerschichten dieser Zweischicht-Hohlmembran in Figur 6 dargestellt.

Es entstand eine erfindungsgemäße Zweischicht-Hohlmembran aus einer amingruppenhaltigen inneren Schicht, die z.B. mit Heparin leicht blutverträglich funktionalisiert werden kann, und einer gewebeverträglichen, äußeren Schicht. Figur 5 belegt die hohe strukturelle Integrität zwischen beiden Polymerschichten. In Figur 6 ist durch klar differenzierte, strukturelle Unterschiede die Grenzfläche zwischen beiden Polymerschichten eindeutig zu identifizieren. Wiederum erhält man eine praktisch ideale strukturelle Integrität der beiden Polymerschichten des Membranquerschnittes.

Weitere charakteristische Daten dieser Hohlmembran sind im folgendem zusammengestellt:

| | |
|---|---|
| Innendurchmesser [µm] | :530 |
| Gesamtwandstärke [µm] | :76 |
| Wasserdurchlässigkeit [µm⁻²h⁻¹kPa⁻¹] | :0,70 |
| Mittlerer.Porendurchmesser [nm] | :6,4 |
| Cut-off [kDa] | :42,7 |

Die Anwesenheit von Amin-Gruppen in Polymermaterialien kann, dem Fachmann bekannt, durch Färbung mit Trinitrobenzensulfonat (TNBS-Assay; Farbwechsel von weiß nach gelb)) qualitativ nachgewiesen werden. Bei Anwendung dieses Farbtestes veränderte sich die Farbe der inneren Schicht der Zweischicht-Hohlmembran des Beispieles 3 von weiß nach gelborange, was die Anwesenheit von Amin-Funktionen qualitativ belegt. Gleichzeitig ist die intensive Färbung in Richtung gelborange ein Hinweis darauf, dass die frei verfügbaren Amin-Gruppen in wässriger Umgebung reaktiv und folglich für eine Heparinisierung geeignet sind. Freie Amin-Gruppen können, dem Fachmann bekannt, mit Heparin funktionalisiert werden, was zu einer verbesserten Blutverträglichkeit der Polymeren führt (z.B.: K.G. Al-Lamee, Y. Taktak: New methods for surface modification and covalent attachment of heparin. Med. Device Technol. 9 (1998):24-27; W. Marconi, F. Benvenuti, A. Piozzi: Covalent bonding of heparin to a vinyl copolymer for biomedical applications. Biomaterials 18 (1997):885-890; C.H. Bamford, K.G. Al-Lamee: Chemical methods for improving the haemocompatibility of synthetic polymers. Clin. Mater. 10 (1992):243-261).

Zur Heparinisierung wurden die Zweischichtmembranen des Beispieles 3 bei Raumtemperatur 24 h in eine Lösung eingelegt, die 10 mg/ml Heparin und ein Kopplungsagent im Molverhältnis 40:1, bezogen auf Heparin, enthielt. Die heparinisierte und intensiv mit Wasser gewaschene Zweischicht-Hohlmembran wurde anschließend mit Toluidinblau angefärbt, wobei sich die Farbe der inneren Polymerschicht dieser Zweischicht-Hohlmembran von weiß nach dunkelblau veränderte, was qualitativ auf hohe Quantitäten an gebundenen Heparin hinweist.

### Beispiel 4: (Anwendungsbeispiel)

MDCK-Zellen wurden für bis zu 8 Tagen in einem Faser-in-Faser-Bioreaktor (vergl.: EP 1 090 984) zwischen zwei chemisch unterschiedlichen Hohlfasern kultiviert. Die äußere Faser bestand aus hydrophilisiertem Polysulfon (geringe Gewebekompatibilität) mit einem Innendurchmesser von 818 nm. Als innere Faser wurde eine ANNVP 5-Hohlfaser verwendet, deren Gewebeverträglichkeit vergleichbar mit der im Beispiel 2 beschriebene Zweischicht-Hohlfaser ist (50 %iger Anteil dieses Polymers in der Außenschicht).

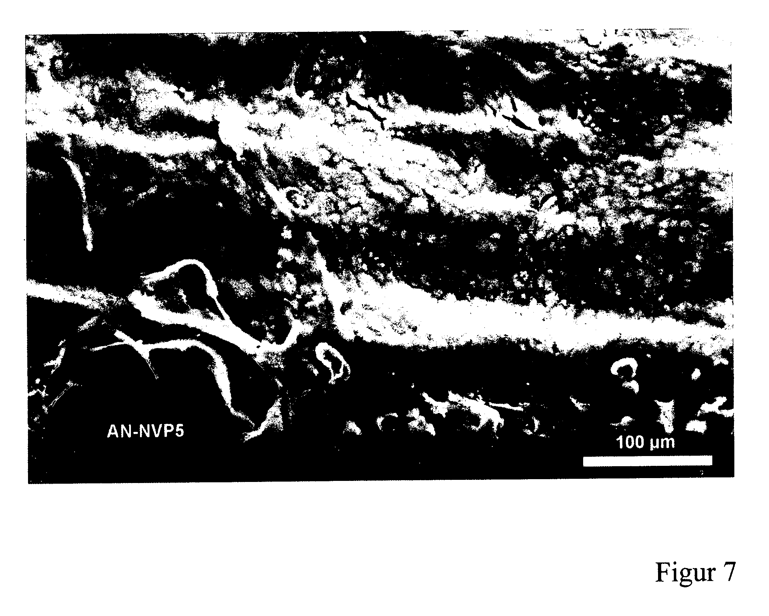

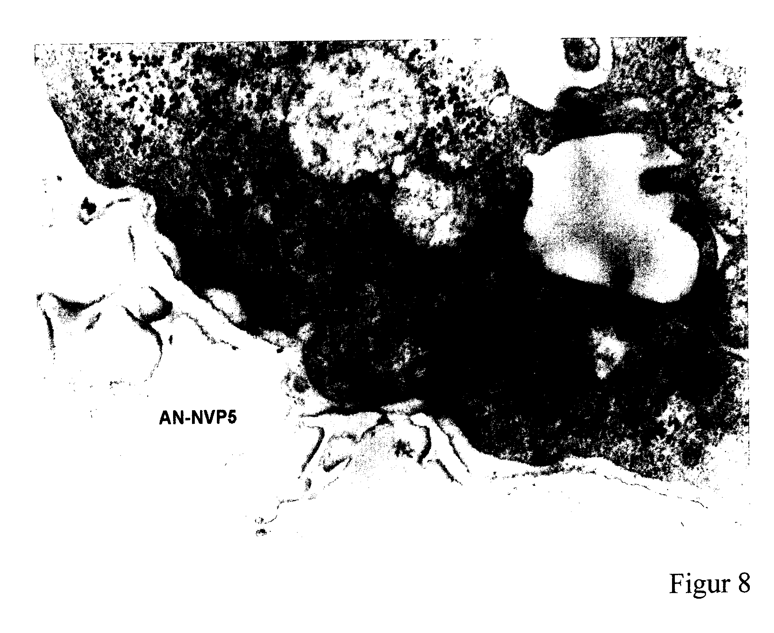

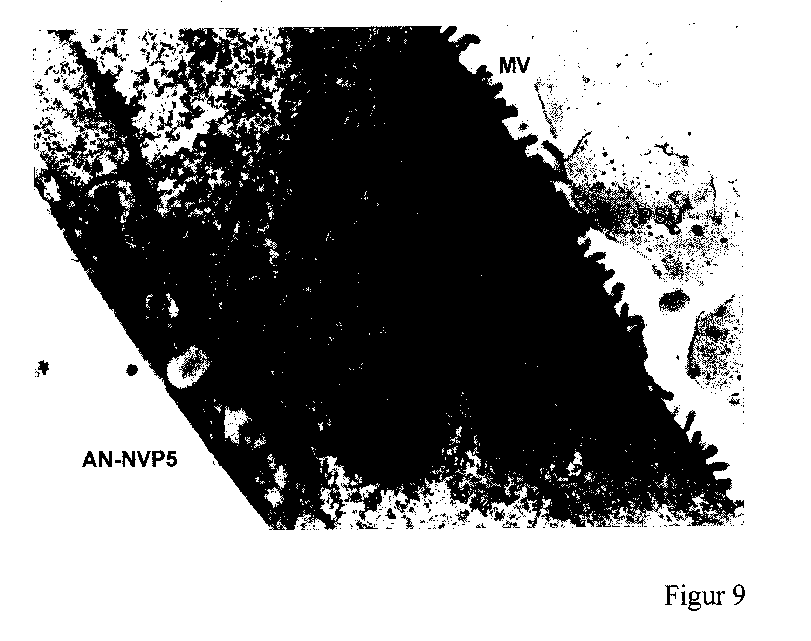

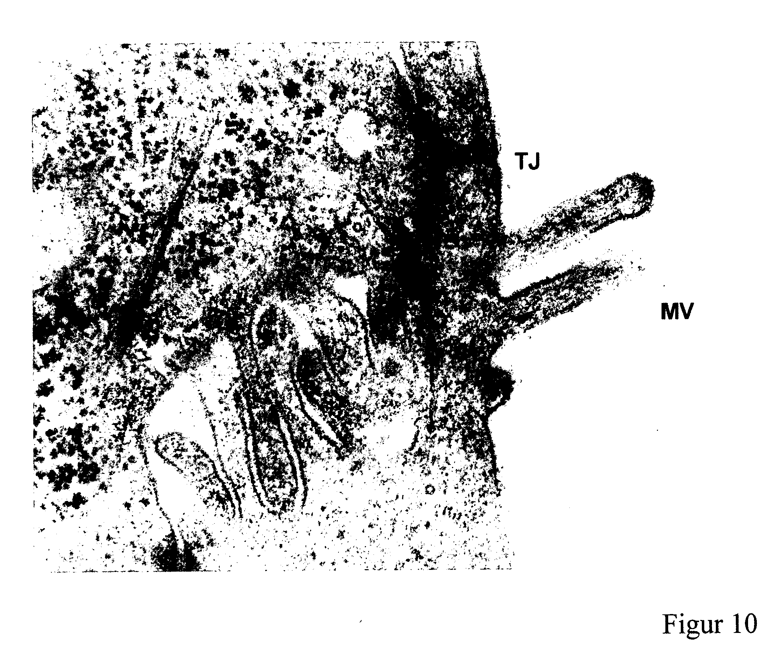

Die Ergebnisse zeigten, dass die MDCK-Zellen vorzugsweise an der inneren Faser adherieren und dort nach 8 Tagen eine konfluente Schicht ebenmäßiger, hexagonaler Zellen ausbilden, die die MDCK-typische Morphologie aufweisen (Figur 7). TEM-Aufnahmen zeigen einen engen Kontakt der Zellen mit der ANNVP 5-Faser (Figur 8). Zahlreiche Mikrovillositäten (MV, Figur 9) sind ebenso wie Zell-Zell-Kontakte (tight junctions - TJ, Figur 10) sichtbar und gut entwickelt. Diese Befunde sprechen für eine intakte Zellschicht, die als funktionstüchtiges Epithel wirken kann, was die hohe Verträglichkeit der Membranoberfläche mit Nierenepithelzellen bestätigt.

## Patentansprüche

1. Trägermembran für Biohybridorgane und Biohybridreaktoren mit einer Zweischicht-Membranwand, deren Schichten aus unterschiedlichen Polymeren bzw. Polymergemischen auf der Basis von Polyacrylnitril bestehen und deren eine Oberfläche blutkompatibel ist, während deren andere Oberfläche gewebekompatibel ist, **dadurch** erhältlich, dass eine erste Polymerlösung, die ein blutkompatibles Polymer enthält, und eine zweite Polymerlösung, die ein gewebekompatibles Polymer enthält, wobei das blutkompatible Polymer das Polyacrylnitril-Copolymer P(AN-co-NVP) mit einem Anteil von 18,0 Mol-% NVP oder heparinisiertes P(AN-co-APMA) mit einem Anteil von 3,8 Mol-% APMA oder eine Mischung von P(AN-co-NVP) mit einem Anteil von 18,0 Mol-% NVP mit Polyacrylnitril ist, wobei das gewebekompatible Polymer das Polyacrylnitril-Copolymer P(AN-co-NVP) mit einem Anteil von 6,1 Mol-% NVP oder eine Mischung von P(AN-co-NVP) mit einem Anteil von 6,1 Mol-% NVP und Polyacrylnitril ist, getrennt voneinander durch Lösen des jeweiligen Polymers in einem Lösemittel derart hergestellt werden, dass beide Polymerlösungen in Form von Polymermischlösungen im homogenen Zustand verbleiben, die beiden Polymerlösungen in der Düse eines Extruders miteinander unter Bildung eines schichtartigen Polymerlösungsverbundes in Kontakt gebracht werden und gleichzeitig in dieser Form als Polymerlösungsverbund aus der Düse des Extruders extrudiert werden, der Polymerlösungsverbund in ein Koagulationsbad extrudiert und einer Phaseninversion unterzogen wird und die gebildete Zweischicht-Membran auf per se bekannte Weise von allen nichtmembranbildenden Bestandteilen zumindest teilweise befreit und erforderlichenfalls nachbehandelt wird.

2. Trägermembran nach Anspruch 1, **dadurch** erhältlich, dass vor der Extrusion in das Koagulationsbad eine Luftstrecke dazwischengeschaltet wird.

3. Trägermembran nach Anspruch 1 oder 2 in Form einer Zweischicht-Hohlmembran, **dadurch** erhältlich, dass als Extruder eine Spinndüse eingesetzt wird.

4. Trägermembran nach Anspruch 3, **dadurch** erhältlich, dass als Spinndüse eine Mehrkanalhohlkerndüse eingesetzt wird.

5. Trägermembran nach Anspruch 3 oder 4, **dadurch** erhältlich, dass der Polymerlösungsverbund am Ausgang der Spinndüse durch die gleichzeitige Extrusion eines den Hohlkern bildenden Lumenfüllers in seiner Form stabilisiert wird.

6. Trägermembran nach einem der Ansprüche 3 bis 5, **dadurch gekennzeichnet, dass** die innere Oberfläche der Hohlmembran blutkompatibel und die äußere Oberfläche gewebekompatibel ist.

7. Trägermembran nach einem der vorhergehenden Ansprüche, **dadurch** erhältlich, dass eine erste und eine zweite Polymerlösung eingesetzt werden, die das gleiche Lösemittel enthalten.

8. Trägermembran nach einem der vorhergehenden Ansprüche, **dadurch** erhältlich, dass eine erste und eine zweite Polymerlösung eingesetzt werden, die ein zusätzliches Polymer enthalten, das insbesondere in beiden Polymerlösungen das gleiche ist.

9. Trägermembran nach Anspruch 8, **dadurch** erhältlich, dass der Massenteil an blutkompatiblen bzw. gewebeverträglichen Polymeren und dem zusätzlichen Polymer im Gesamtpolymeranteil der jeweiligen Polymerlösung 10 bis 90 und insbesondere 40 bis 60 % beträgt.

10. Trägermembran nach einem der vorhergehenden Ansprüche, **dadurch** erhältlich, dass die Konzentration an Polymer in der ersten und zweiten Polymerlösung jeweils 10 bis 30 % beträgt.

11. Verfahren zur Herstellung einer Trägermembran nach einem der vorhergehenden Ansprüche, **dadurch gekennzeichnet, dass** die in den vorhergehenden Ansprüchen beschriebenen Verfahrensmaßnahmen durchgeführt werden.

12. Verwendung einer Trägermembran nach einem der Ansprüche 1 bis 10 als Trägermembran für Biohybridorgane.

## Claims

1. A support membrane for biohybrid elements and biohybrid reactors with a double-layer membrane wall, the layers of which consist of different polymers or polymer mixtures based on polyacrylonitrile and one surface of which is blood compatible whilst their other surface is tissue compatible, which is obtainable by producing a first polymer solution, which contains a blood compatible polymer, and a second polymer solution, which contains a tissue compatible polymer, wherein the blood compatible polymer is the polyacrylonitrile-copolymer P(AN-co-NVP) with a content of 18.0 mol% NVP or heparinised P(AN-co-APMA) with a content of 3.8 mol% APMA or a mixture of P(AN-co-NVP) with a content of 18.0 mol% NVP with polyacrylonitrile, wherein the tissue compatible polymer is the polyacrylonitrile copolymer P(AN-co-NVP) with a content of 6.1 mol% NVP or a mixture of P(AN-co-NVP) with a content of 6.1 mol% NVP and polyacrylonitrile, separately from one another by dissolving each polymer in a solvent such that both polymer solutions remain in the form of polymer mixed solutions in a homogenous state, bringing the two polymer solutions into contact with one another in the nozzle of an extruder whilst forming a layer-like polymer solution composite and simultaneously extruding it in this form out of the nozzle of the extruder in the form of a polymer solution composite, extruding the polymer solution composite into a coagulation bath and subjecting it to a phase inversion and at least partially freeing the double layer membrane formed of all non-membrane forming components in a manner known per *se* and, if necessary, post-treating it.

2. A support membrane as claimed in Claim 1, obtainable by interposing an air gap before the extrusion into the coagulation bath.

3. A support membrane as claimed in Claim 1 or 2 in the form of a double layer hollow membrane, obtainable by using a spinning nozzle as the extruder.

4. A support membrane as claimed in Claim 3, obtainable by using a multichannel hollow core nozzle as the spinning nozzle.

5. A support membrane as claimed in Claim 3 or 4, obtainable by stabilising the polymer composite compound in its shape at the outlet of the spinning nozzle by the simultaneous extrusion of a lumen filler constituting the hollow core.

6. A support membrane as claimed in one of Claims 3 to 5, **characterised in that** the inner surface of the hollow membrane is blood compatible and the outer surface is tissue compatible.

7. A support membrane as claimed in one of the preceding claims, obtainable by using first and second polymer solutions, which contain the same solvent.

8. A support membrane as claimed in one of the preceding claims, obtainable by using first and second polymer solutions, which contain an additional polymer, which, in particular, is the same in both polymer solutions.

9. A support membrane as claimed in Claim 8, obtainable by the mass proportion of blood compatible or tissue compatible polymers and the additional polymer in the overall polymer content of each polymer solution being 10 to 90 and particularly 40 to 60%.

10. A support membrane as claimed in one of the preceding claims, obtainable by the concentration of polymer in the first and second polymer solutions being 10 to 30% in each case.

11. A method of producing a support membrane as claimed in one of the preceding claims, **characterised in that** the method steps described in the preceding claims are performed.

12. Use of a support membrane as claimed in one of Claims 1 to 10 as a support membrane for biohybrid elements.

## Revendications

1. Membrane-support pour organes biohybrides et réacteurs biohybrides comportant une paroi de membrane à deux couches dont les couches se composent de différents polymères ou mélanges de polymères sur une base de polyacrylonitrile et dont une surface est compatible avec le sang, tandis que l'autre surface est compatible avec les tissus, pouvant être obtenue en ce qu'une première solution polymère qui contient un polymère compatible avec le sang et une seconde solution polymère qui contient un polymère compatible avec les tissus, le polymère compatible avec le sang étant le copolymère de polyacrylonitrile P(AN-co-NVP) présentant une fraction de 18,0 % en moles de NVP, ou le P(AN-co-APMA) héparinisé présentant une fraction de 3,8 % en moles d'APMA, ou un mélange de P(AN-co-NVP) présentant une fraction de 18,0 % en moles de NVP avec du polyacrylonitrile, et le polymère compatible avec les tissus étant le copolymère de polyacrylonitrile P(AN-co-NVP) présentant une fraction de 6,1 % en moles de NVP ou un mélange de P(AN-co-NVP) présentant une fraction de 6,1 % en moles de NVP et de polyacrylonitrile, sont fabriquées séparément par dissolution des polymères respectifs dans un solvant de façon à ce que les deux solutions polymères restent à l'état homogène sous la forme de solutions de mélanges polymères, en ce que les deux solutions polymères sont mises en contact l'une avec l'autre dans la buse d'une extrudeuse en formant un composite de solution polymère de type à couches, et sont extrudées simultanément sous cette forme, en tant que composite de solution polymère, hors de la buse de l'extrudeuse, en ce que le composite de solution polymère est extrudé dans un bain de coagulation et est soumis à une inversion de phase, et en ce que la membrane à deux couches formée est débarrassée au moins partiellement, de façon connue en soi, de tous les composants ne formant pas la membrane et est soumise le cas échéant à un traitement ultérieur.

2. Membrane-support selon la revendication 1, pouvant être obtenue en ce qu'avant l'extrusion, dans le bain de coagulation, on intercale une distance dans l'air.

3. Membrane-support selon la revendication 1 ou 2 sous la forme d'une membrane creuse à deux couches, pouvant être obtenue en ce que l'on utilise comme extrudeuse une filière.

4. Membrane-support selon la revendication 3, pouvant être obtenue en ce que l'on utilise comme filière une filière multicanaux à noyau creux.

5. Membrane-support selon la revendication 3 ou 4, pouvant être obtenue en ce que le composite de solution polymère en sortie de la filière est stabilisé dans sa forme par l'extrusion simultanée d'une matière de charge de lumen formant le noyau creux.

6. Membrane-support selon l'une des revendications 3 à 5, **caractérisée en ce que** la surface interne de la membrane creuse est compatible avec le sang et la surface externe est compatible avec les tissus.

7. Membrane-support selon l'une des revendications précédentes, pouvant être obtenue en ce que l'on utilise une première et une seconde solutions polymères qui contiennent le même solvant.

8. Membrane-support selon l'une des revendications précédentes, pouvant être obtenue en ce que l'on utilise une première et une seconde solutions polymères qui contiennent un polymère supplémentaire qui est en particulier le même dans les deux solutions polymères.

9. Membrane-support selon la revendication 8, pouvant être obtenue en ce que la fraction en masse de polymères compatibles avec le sang ou compatibles avec les tissus et de polymère supplémentaire par rapport à la fraction totale des polymères de la solution polymère respective est comprise entre 10 et 90 %, et en particulier entre 40 et 60 %.

10. Membrane-support selon l'une des revendications précédentes, pouvant être obtenue en ce que la concentration en polymère dans la première et dans la seconde solutions polymères est respectivement comprise entre 10 et 30 %.

11. Procédé de fabrication d'une membrane-support selon l'une des revendications précédentes, **caractérisé en ce que** l'on met en oeuvre les modes opératoires décrits dans les revendications précédentes.

12. Utilisation d'une membrane-support selon l'une des revendications 1 à 10 en tant que membrane-support pour organes biohybrides.
